(19) **Europäisches Patentamt / European Patent Office / Office européen des brevets**

(11) **EP 2 985 592 B1**

(12) **EUROPÄISCHE PATENTSCHRIFT**

(45) Veröffentlichungstag und Bekanntmachung des Hinweises auf die Patenterteilung:
**27.09.2017 Patentblatt 2017/39**

(51) Int Cl.:
**G01N 21/3504** *(2014.01)* **G01J 3/433** *(2006.01)*
**G01N 21/39** *(2006.01)* **G01N 21/31** *(2006.01)*

(21) Anmeldenummer: **14180764.4**

(22) Anmeldetag: **13.08.2014**

(54) **Absorptionsspektrometer und Verfahren zur Messung der Konzentration einer interessierenden Gaskomponente eines Messgases**

Absorption spectrometer and method for measuring the concentration of an interesting gas component of a measuring gas

Spectromètre à absorption et procédé destiné à mesurer la concentration d'un composant gazeux pertinent d'un gaz de mesure

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**17.02.2016 Patentblatt 2016/07**

(73) Patentinhaber: **Siemens Aktiengesellschaft 80333 München (DE)**

(72) Erfinder: **Bitter, Ralf 76139 Karlsruhe (DE)**

(56) Entgegenhaltungen:
JP-A- H0 579 976 JP-A- 2004 361 129
US-A- 5 173 749 US-A1- 2008 123 712
US-A1- 2012 188 549 US-A1- 2013 163 000

EP 2 985 592 B1

**Beschreibung**

Die Erfindung betrifft ein Absorptionsspektrometer zur Messung der Konzentration einer interessierenden Gaskomponente eines Messgases, mit

- einem wellenlängendurchstimmbaren Laser,
- einem Detektor zur Erzeugung eines Messsignals,
- einer im Lichtweg zwischen dem Laser und dem Detektor liegenden und das Messgas enthaltenden Multireflexions-Gaszelle,
- Steuermitteln zur Variation des Injektionsstroms des Lasers, um die Wellenlänge des Lichts auf eine spezifische Absorptionslinie der interessierenden Gaskomponente abzustimmen, und
- Auswertemitteln zur Auswertung des Messsignals und Ermittlung eines Messergebnisses für die zu messende Konzentration.

Die Erfindung betrifft ferner ein Verfahren zur Messung der Konzentration einer interessierenden Gaskomponente eines Messgases, wobei

- die Wellenlänge des Lichts eines Lasers auf eine spezifische Absorptionslinie der interessierenden Gaskomponente abgestimmt wird,
- das modulierte Licht durch eine das Messgas enthaltende Multireflexions-Gaszelle auf einen Detektor geführt wird und
- ein von dem Detektor erzeugtes Messsignal zur Ermittlung eines Messergebnisses für die zu messende Konzentration ausgewertet wird.

Ein derartiges Absorptionsspektrometer bzw. Verfahren sind beispielsweise jeweils aus der WO 2012/109030 A1 und der US 5 173 749 A bekannt.

Laserspektrometer werden für die optische Gasanalyse, z. B. in der Prozessmesstechnik, eingesetzt. Dabei erzeugt ein Laser, in der Regel eine Laserdiode, Licht im Infrarotbereich, das durch ein zu messendes Messgas (Prozessgas) geführt und anschließend detektiert wird. Die Wellenlänge des Lichts wird auf eine spezifische Absorptionslinie der jeweils zu messenden Gaskomponente abgestimmt, wozu die Laserdiode üblicherweise periodisch mit einem rampen- oder dreieckförmigen Stromsignal angesteuert wird, um die Absorptionslinie mit dem erzeugten Licht periodisch wellenlängenabhängig abzutasten.

Bei der direkten Absorptionsspektroskopie wird die Konzentration der interessierenden Gaskomponente unmittelbar aus der an der Stelle der Absorptionslinie detektierten Minderung der Lichtintensität (Absorption) bestimmt. Bei der Wellenlängen-Modulationsspektroskopie wird während der vergleichsweise langsamen wellenlängenabhängigen Abtastung der Absorptionslinie zusätzlich die Wellenlänge des erzeugten Lichts mit einer vergleichsweise hohen Frequenz im kHz-Bereich und mit kleiner Amplitude sinusförmig moduliert. Da das Profil der Absorptionslinie nicht linear ist, werden Harmonische höherer Ordnung in dem Detektor- oder Messsignal erzeugt. Das Messsignal wird bei einer solchen höheren Harmonischen, vorzugsweise der zweiten Harmonischen, durch phasensensitive Lock-in Technik demoduliert und für jedes Abtastintervall zu einem Messergebnis ausgewertet. Die Auswertung erfolgt beispielsweise durch Anfitten (Curve-Fitting) des im Idealfall zu erwartenden und mittels eines Näherungsmodells analytisch beschriebenen Verlaufs des demodulierten Messsignals (Sollkurve) an dessen tatsächlichen Verlauf (Istkurve). Da einer der Parameter des Näherungsmodells zu der Konzentration der Gaskomponente proportional ist, erhält man als Ergebnis der Auswertung und damit als Messergebnis die Konzentration der zu messenden Gaskomponente.

Die Wellenlängen-Modulationsspektroskopie ist insbesondere bei der Messung geringer Konzentrationen im Vorteil, weil sie Rauschen aus dem Messsignal besser ausfiltern kann. Bei höheren Konzentrationen werden jedoch die für die Auswertung des Messsignals notwendigen Näherungen zunehmend ungenau, wodurch der Messfehler steigt. Bei der Absorptionsspektroskopie ist dagegen der Messfehler wegen der höheren Rauschempfindlichkeit bei kleinen Konzentrationen höher. Da aber keine Näherungsbeschreibung der Absorptionslinie notwendig ist, wird die Messgenauigkeit mit zunehmender Konzentration besser, weil dann auch das Nutzsignal stärker wird.

Die Größe des Messsignals ist umgekehrt proportional zu der Absorption des Lichts auf dem Weg von dem Laser zum Detektor. Die Absorption an der Stelle der spezifischen Absorptionslinie ist ihrerseits gemäß dem Lambert-Beer'schen Gesetz monoton abhängig, bei den meisten Messaufgaben näherungsweise proportional, zu dem Produkt aus der Konzentration der interessierenden Gaskomponente und der Länge des Lichtwegs zwischen dem Laser und dem Detektor. Je geringer die zu messenden Konzentrationen sind, umso länger muss die Absorptionsstrecke sein, um ein ausreichend großes Messsignal zu erhalten. Während bei in-situ Messungen in der Regel aufgrund der baulichen Gegebenheiten der Prozessanlage lange Absorptionsstrecken gegeben sind (z. B. Schornstein in einer Verbrennungsanlage), besteht für extraktive Messungen, bei denen das Messgas durch eine zwischen dem Laser und dem Detektor liegende Gaszelle geleitet wird, die Herausforderung, eine lange Absorptionsstrecke auf kleinem Raum zu realisieren. Dies geschieht üblicherweise mittels einer Multireflexions-Gaszelle, wie z. B. einer Herriott- oder White-Zelle, in der die optische Weglänge und damit die Absorptionsstrecke durch mehrfache Reflexion des Lichts zwischen Spiegeln vergrößert ist. Eine Schwäche solcher Gaszellen ist jedoch ihre Empfindlichkeit in Bezug auf Umgebungseinflüsse wie Temperaturschwankungen oder Erschütterungen. So können relativ kleine Änderungen der geometrischen Parameter, wie der Einstrahlwinkel des Lasers oder die Winkel und Abstände der Spiegel, zu größeren Änderungen der optischen Weglänge und somit der Absorptionsstrecke führen, insbesondere, wenn dadurch die Anzahl der Reflexionen variiert.

Aus der eingangs genannten US 5 173 749 A ist es bekannt, das Licht des Lasers auf einen Messpfad durch die Multireflexions-Gaszelle zu dem Messdetektor und parallel dazu auf einen Referenzpfad durch eine Referenz-Gaszelle zu einem Referenzdetektor aufzuteilen. Aus der Phasendifferenz zwischen dem demodulierten Messsignal des Messdetektors und dem demodulierten Referenzsignal des Referenzdetektors kann bei bekannter Länge des Referenzpfades die Länge des Messpfades berechnet werden.

Der Erfindung liegt daher die Aufgabe zugrunde, den Einfluss von Änderungen der optischen Weglänge in dem Absorptionsspektrometer auf das Messergebnis zu kompensieren.

Gemäß der Erfindung wird die Aufgabe durch das in Anspruch 1 angegebene Absorptionsspektrometer oder das in Anspruch 3 angegebene Verfahren gelöst.

Vorteilhafte Weiterbildungen des erfindungsgemäßen Absorptionsspektrometers bzw. Verfahrens sind in den Unteransprüchen angegeben.

Entsprechend der Erfindung wird dass das Licht des Lasers über dessen Injektionsstrom mit mindestens einer zwei Pilotfrequenzen im MHz-Bereich moduliert. In dem Messsignal enthaltene Signalanteile mit den Pilotfrequenzen werden jeweils phasensensitiv ausgewertet. Eine Differenz der dabei erhaltenen Phaseninformationen beider Signalanteile kann mit einer bei Kalibration des Absorptionsspektrometers erhaltenen Differenz der Phaseninformationen verglichen werden, um in Abhängigkeit von dem Verhältnis beider Differenzen das Messergebnis zu korrigieren.

Die Phasen der in dem Messsignal enthaltenen Signalanteile mit den zwei Pilotfrequenzen sind von der Laufzeit des Lichts von dem Laser zu dem Detektor und damit von der optischen Wegstrecke abhängig. Aufgrund der Mehrfachreflexionen in der Multireflexions-Gaszelle ist angesichts der kurzen Wegstrecken zwischen der Gaszelle und dem Laser bzw. dem Detektor die Absorptionsstrecke weitgehend identisch mit der optischen Wegstrecke. Den Pilotfrequenzen im MHz-Bereich entspricht eine Wellenlänge im Meterbereich, so dass Änderungen der optischen Weglänge sicher über die Phaseninformationen erfasst werden können.

Aufgrund von Störungen kann sich die optische Weglänge nicht nur zwischen aufeinander folgenden Messungen sondern auch innerhalb jeder Messperiode ändern. Da die Änderungen der optischen Weglänge jedoch unmittelbar und gleichzeitig mit der Messung der Absorption erfasst werden, können die erfassten optischen Weglängenänderungen für die Korrektur des Messergebnisses gemittelt werden.

Das Messergebnis, also die Konzentration der interessierenden Gaskomponente, bestimmt sich aus der an der Stelle der Absorptionslinie detektierten Absorption und der Länge der Absorptionsstrecke. Die von Stör- und Umwelteinflüssen freie Absorptionsstrecke ist entweder bekannt, sie kann gemessen werden oder sie wird implizit bei der Kalibration des Absorptionsspektrometers erfasst, wenn bei unterschiedlichen bekannten Konzentrationswerten unterschiedliche Absorptionswerte gemessen und zusammen mit den zugehörigen Konzentrationswerten abgespeichert werden.

Die Verwendung zweier Pilotfrequenzen und Auswertung der Differenz der erhaltenen Phaseninformationen hat den Vorteil, dass bei der Signalerzeugung und Signalauswertung vorkommende und sich bei beiden Frequenzen gleichermaßen auswirkende Phasenfehler oder -offsets gegenseitig aufheben. Außerdem besteht, wie später noch erläutert wird, ein größerer Freiraum für die Wahl geeigneter Frequenzwerte.

Aus der Differenz der erhaltenen Phaseninformationen beider Signalanteile kann auch die Länge des Lichtwegs bestimmt und mit einer bei Kalibration des Absorptionsspektrometers erhaltenen Länge oder der bekannten Länge des Lichtwegs verglichen werden. In Abhängigkeit von dem Verhältnis beider Längen wird das Messergebnis korrigiert.

Hierbei besteht der Vorteil darin, dass die momentane (Ist-) Länge des Lichtwegs über die Differenz der erhaltenen Phaseninformationen unmittelbar gemessen wird, so dass eine einfache Korrektur des Messergebnisses in Bezug auf die bekannte oder durch Kalibration ermittelte Soll-Länge des Lichtwegs möglich ist.

Mit der aus der Differenz der erhaltenen Phaseninformationen beider Signalanteile bestimmten Länge kann schließlich aus der von dem Messsignal repräsentierten Absorption unmittelbar das Messergebnis ermittelt werden.

Bei direkter Messung der Absorptionsminderung, also im Falle der direkten Absorptionsspektroskopie, ist es grundsätzlich möglich, die korrekte Konzentration der interessierenden Gaskomponente unmittelbar aus der Absorption zu bestimmen. Da die momentane Länge des Lichtwegs über die Differenz der erhaltenen Phaseninformationen unmittelbar gemessen wird, kann anschließend aus der von dem Messsignal unmittelbar das Messergebnis für die zu messende Konzentration werden.

Bei Betrieb des erfindungsgemäßen Absorptionsspektrometers auf Basis der Wellenlängen-Modulationsspektroskopie kann in vorteilhafter Weise eine der Pilotfrequenzen als Modulationsfrequenz verwendet werden, wobei in an sich bekannter Weise das Messsignal bei einer höheren Harmonischen, insbesondere der zweiten Harmonischen, der als Modulationsfrequenz verwendeten Pilotfrequenz demoduliert und zur Ermittlung des Messergebnisses ausgewertet wird.

Generell sind die die Pilotfrequenzen so niedrig zu wählen, dass bei den zu erwartenden bzw. maximal tolerierbaren Änderungen der optischen Weglänge Phasengänge größer als n vermieden werden, so dass die erhaltenen Phaseninformationen eindeutig sind. Bei Modulation des Lichts mit und phasensensitiver Auswertung des Messsignals bei zwei Pilotfrequenzen ergibt sich die vorteilhafte Möglichkeit, die eine Pilotfrequenz so hoch gewählt wird, dass sich die daraus erhaltene Phaseninformationen bei einer maximal tolerierbaren Änderung der optischen Wegstrecke zwischen dem Laser und dem Detektor um mehr als $k \cdot \pi$ ($k \geq 1$) ändert, und die andere Pilotfrequenz so niedrig gewählt wird, dass sich die daraus erhaltene Phaseninformation bei der maximal tolerierbaren Änderung der optischen Wegstrecke um weniger als n ändert. Anhand der Phaseninformation aus der niedrigeren Pilotfrequenz kann dann der Wert k in der Phaseninformation aus der höheren Pilotfrequenz bestimmt werden. Auf diese Weise wird durch die Auswertung des Messsignals bei der höheren Pilotfrequenz eine hohe Winkelauflösung erreicht, wobei die damit verbundene Mehrdeutigkeit mittels der Phaseninformation aus der niedrigeren Pilotfrequenz wieder in eine Eindeutigkeit zu gewandelt wird.

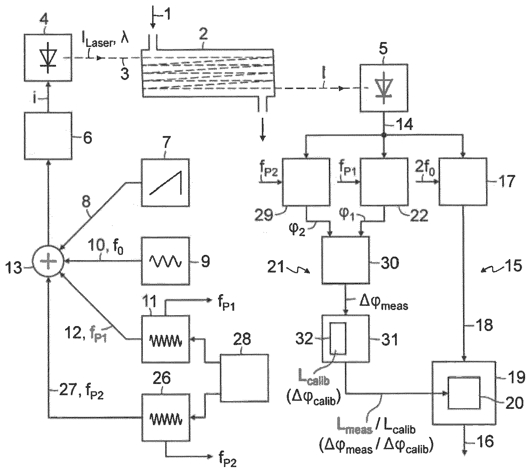

Zur weiteren Erläuterung der Erfindung wird im Folgenden auf die einzige Figur der Zeichnung Bezug genommen, die ein Ausführungsbeispiel des erfindungsgemäßen Absorptionsspektrometers zeigt.

Die Figur zeigt in Form eines vereinfachten Blockschaltbildes ein auf der Basis der Wellenlängen-Modulationsspektroskopie (WMS) arbeitendes Absorptionsspektrometer zur Messung der Konzentration mindestens einer interessierenden Gaskomponente eines Messgases 1, welches durch eine Multireflexions-Gaszelle 2 geleitet wird. Die Multireflexions-Gaszelle 2 ist in dem Lichtweg 3 zwischen einem Laser 4, hier einer Laserdiode, und einem Detektor 5 angeordnet. Das von dem Laser 4 kommende Licht wird in der Multireflexions-Gaszelle 2 vielfach reflektiert, bevor es auf den Detektor 5 fällt. Bei der Multireflexions-Gaszelle 2 handelt es sich beispielsweise um eine Herriott- oder White-Zelle, so dass ihr tatsächlicher Aufbau von der hier stark schematisierten Darstellung erheblich abweichen kann. Die Laserdiode 4 wird von einer steuerbaren Stromquelle 6 mit einem Injektionsstrom i angesteuert, wobei die Intensität $I_{Laser}$ und Wellenlänge $\lambda$ des erzeugten Lichts von dem Strom i und der Betriebstemperatur der Laserdiode 4 abhängen. Die Stromquelle 6 wird von einem ersten Signalgenerator 7 periodisch mit einer vorgegebenen, vorzugsweise rampen- oder dreieckförmigen Funktion 8 angesteuert, um mit der mehr oder weniger linear dem Verlauf des Stromes i folgenden Wellenlänge $\lambda$ des erzeugten Lichts eine ausgewählte Absorptionslinie der interessierenden Gaskomponente abzutasten. Ein zweiter Signalgenerator 9 erzeugt ein sinusförmiges Modulationssignal 10 mit einer Modulationsfrequenz $f_0$ im kHz-Bereich, ein dritter Signalgenerator (erster Pilotsignalgenerator) 11 ein sinusförmiges Pilotsignal 12 mit einer Pilotfrequenz $f_{P1}$ im MHz-Bereich und ein vierter Signalgenerator (zweiter Pilotsignalgenerator) 26 ein zweites sinusförmiges Pilotsignal 27 mit einer zweiten Pilotfrequenz $f_{P2}$, die ebenfalls im MHz-Bereich liegt, aber von der ersten Pilotfrequenz $f_{P1}$ verschieden ist. Mit diesen Signalen 10, 12, 27 wird die rampen- oder dreieckförmige Funktion 8 in einem Summierglied 13 moduliert.

Bei dem gezeigten Ausführungsbeispiel werden die beiden Pilotsignale 12, 27 gleichzeitig erzeugt. Sie können aber auch abwechselnd von beiden Pilotsignalgeneratoren 11, 26 oder von nur einem Pilotsignalgenerator, z. B 11, durch Verändern der Pilotfrequenz $f_P$ unter Beibehaltung der Phasenlage erzeugt werden. Zu diesem Zweck können die Pilotsignalgeneratoren 11, 26 von einer Synchronisiereinrichtung 28 entsprechend angesteuert werden.

Der Detektor 5 erzeugt in Abhängigkeit von der detektierten Lichtintensität I ein Detektor- oder Messsignal 14, das in Auswertemitteln 15 zur Ermittlung eines Messergebnisses 16 für die zu messende Konzentration der interessierenden Gaskomponente ausgewertet wird. Wegen des nicht linearen Profils der abgetasteten Absorptionslinie führt die Modulation des Stroms i der Laserdiode 4 mit der Modulationsfrequenz $f_0$ zu eine entsprechenden Variation der detektierten Lichtintensität I mit mehr oder weniger starken harmonischen Verzerrungen. Da die zweite Harmonische mit der Frequenz $2f_0$ an der Stelle des Absorptionsmaximums dominiert und in Wellenlängenbereichen außerhalb der Mitte der Absorptionslinie stark abnimmt, ist sie für die weitere Auswertung besonders geeignet. Prinzipiell kann auch jede andere Harmonische zur Auswertung verwendet werden. Bei dem gezeigten Ausführungsbeispiel wird das Messsignal 14 in einem ersten Lock-in-Verstärker 17 bei der zweiten Harmonischen $2f_0$ der Modulationsfrequenz $f_0$ phasensensitiv demoduliert. Das demodulierte Messsignal 18 wird in einer Auswerteeinrichtung 19 für jedes Abtastintervall zu dem Messergebnis 16 ausgewertet. Die Auswertung erfolgt hier z. B. durch Anfitten des in einem Näherungsmodell 20 analytisch beschriebenen idealen Verlaufs des demodulierten Messsignals an den Verlauf des gemessenen demodulierten Messsignals 16. Das Näherungsmodell 20 enthält eine Vielzahl von Parametern, darunter auch einen Parameter für die Länge der Absorptionsstrecke in der Multireflexions-Gaszelle 2 und einen Parameter für die Konzentration der zu bestimmenden Gaskomponente. Über den letztgenannten Parameter erhält man als Ergebnis der Auswertung und damit als Messergebnis die Konzentration der zu messenden Gaskomponente.

Weitere Auswertemittel 21 dienen dazu, die in dem Messsignal 14 enthaltenen Signalanteile mit den Pilotfrequenzen $f_{P1}$, $f_{P2}$ jeweils phasensensitiv auszuwerten. Dazu wird das Messsignal 14 in einem zweiten Lock-in-Verstärker oder Phasendetektor 22 an der Stelle der Pilotfrequenz $f_{P1}$ und in einem dritten Lock-in-Verstärker oder Phasendetektor 29 an der Stelle der Pilotfrequenz $f_{P2}$ in an sich bekannter Weise phasensensitiv detektiert. Dabei wird das Messsignal 14 durch Multiplikation mit einem von dem Pilotsignalgenerator 11 gelieferten Referenzsignal mit der Pilotfrequenz $f_{P1}$ oder direkt mit dem Pilotsignal 12 phasensensitiv demoduliert. Durch anschließende Tiefpassfilterung erhält man die Inphasenkomponente I des demodulierten Messsignals. Bei einem Zweiphasen-Lock-In-Verstärker wird das Messsignal 14 zusätzlich mit dem um 90° phasenverschobenen Referenzsignal 23 multipliziert und durch Tiefpassfilterung. Durch anschließende Tiefpassfilterung erhält man die Quadraturkomponente Q des demodulierten Messsignals. Die Phase $\varphi_1$ des Messsignals ergibt sich dann mit $\varphi_1$ = arctan (Q/I). Es ist auch möglich durch Rückkopplung die Phase bei der Erzeugung des Referenzsignals 23 oder Pilotsignal 12 in dem Pilotsignalgenerator 11 so zu verschieben, dass die Quadraturkomponente zu Null wird, wo bei dann die Phasenverschiebung der gesuchten Phaseninformation $\varphi_1$ entspricht. Auf analoge Weise wird die Phaseninformationen $\varphi_2$ erhalten. Wird, wie oben erwähnt, die Pilotfrequenz $f_P$ unter Beibehaltung der Phasenlage zwischen zwei Werten umgeschaltet, dann werden die beiden Phaseninformationen durch Zeitdemultiplex bestimmt. In einer ersten Recheneinheit 30 wird die Differenz $\Delta\varphi_{meas}$ der gemessenen Phaseninformationen $\varphi_1$, $\varphi_2$ bestimmt. Wie unten noch näher erläutert wird, kann in einer zweiten Recheneinheit 31 aus dieser Differenz $\Delta\varphi_{meas}$ die momentane optischen Weglänge bzw. momentane Länge der Absorptionsstrecke $L_{meas}$ berechnet werden. Da, wie oben bereits erwähnt, das Näherungsmodell 20 einen Parameter für die Länge der Absorptionsstrecke enthält, kann in der Auswerteeinrichtung 19 das Messergebnis 18 unmittelbar in Abhängigkeit von der aktuell gemessenen Länge der Absorptionsstrecke $L_{meas}$ korrigiert werden.

Bei kleinen Konzentrationen der zu messenden Gaskomponente ist die Absorption näherungsweise proportional zu dem Produkt aus dem Konzentrationswert und der Länge der Absorptionsstrecke. D. h., für eine bei Kalibration des Absorptionsspektrometers gemessene Absorption A gilt:

$$A = 1 - e^{-a \cdot c_{calib} \cdot L_0} \approx a \cdot c_{calib} \cdot L_0 \qquad \text{Gl. 1,}$$

wobei

a der     Absorptionskoeffizient der bei der Kalibration verwendeten Gaskomponente,
$c_{calib}$     die bekannte Konzentration der Gaskomponente und
$L_0$     die bekannte Absorptionsstrecke ohne Störeinflüsse sind.

Für die Länge L der Absorptionsstrecke gilt:

$$L = \left(n + \frac{\varphi_1}{2\pi}\right)\frac{c}{f_{P1}} = \left(m + \frac{\varphi_2}{2\pi}\right)\frac{c}{f_{P2}} \qquad \text{Gl. 2,}$$

wobei

n     die Anzahl der Vollschwingungen des in dem Messsignal 14 enthaltenen Signalanteils mit der ersten Pilotfrequenz $f_{P1}$ während der Laufzeit des Lichts von dem Laser 4 zu dem Detektor 5 ist, und
m     die entsprechende Anzahl der Vollschwingungen des Signalanteils mit der zweiten Pilotfrequenz $f_{P2}$ ist.

Daraus folgt für die gemessene Phasendifferenz:

$$\Delta\varphi_{meas} = \varphi_1 - \varphi_2 = \left(L_{meas}\frac{2\pi f_{P1}}{c} - n \cdot 2\pi\right) - \left(L_{meas}\frac{2\pi f_{P2}}{c} - m \cdot 2\pi\right)$$
$$= L_{meas}\frac{2\pi(f_{P1} - f_{P2})}{c} - (n - m) \cdot 2\pi \qquad \text{Gl. 3.}$$

Bei Kenntnis von n und m erhält man als aktuell gemessene Länge der Absorptionsstrecke:

$$L_{meas} = \frac{c}{f_{P1} - f_{P2}} \cdot \left(\frac{\Delta\varphi_{meas}}{2\pi} + (n - m)\right) \qquad \text{Gl. 4}$$

oder speziell für n = m:

$$L_{meas} = \frac{c}{f_{P1}-f_{P2}} \cdot \frac{\Delta\varphi_{meas}}{2\pi} \qquad \text{Gl. 5.}$$

Der gemessene Konzentrationsmesswert $c_{meas}$ lässt sich nun unter Verwendung von Gleichung G1. 5 wie folgt korrigieren:

$$c_{corr} = c_{meas} \cdot \frac{L_0}{L_{meas}} \qquad \text{Gl. 6,}$$

Die von Stör- und Umwelteinflüssen freie Absorptionsstrecke $L_0$ ist an sich bekannt. Sie kann aber auch bei der Kalibration des Absorptionsspektrometers auf dieselbe Weise wie $L_{meas}$ als $L_{calib}$ gemessen und in einem Speicher 32 der zweiten Recheneinheit 31 abgelegt werden. In diesem Fall können anstelle der gemessenen Absorptionsstrecken $L_{meas}$ und $L_{calib}$ die zugehörigen Differenzen $\Delta\varphi_{meas}$ und $\Delta\varphi_{calib}$ der unter Mess- bzw. Kalibrationsbedingungen gemessenen Phaseninformationen $\varphi_1$, $\varphi_2$ verwendet werden. Aus den Gleichungen Gl. 6 und Gl. 5 folgt nämlich:

$$c_{corr} = c_{meas} \cdot \frac{L_{calib}}{L_{meas}} = c_{meas} \cdot \frac{\Delta\varphi_{calib}}{\Delta\varphi_{meas}} \qquad \text{Gl. 7.}$$

Bei direkter Messung der Absorptionsminderung, also im Falle der direkten Absorptionsspektroskopie, ist es grundsätzlich möglich, die korrekte Konzentration der interessierenden Gaskomponente unmittelbar aus der Absorption A zu bestimmen. Aus den Gleichungen Gl. 1 und Gl. 5 folgt nämlich:

$$c_{meas} = \frac{A}{a \cdot L_{meas}} = \frac{A}{a} \cdot \frac{f_{P1}-f_{P2}}{c} \cdot \frac{2\pi}{\Delta\varphi} \qquad \text{Gl. 8.}$$

Das Messergebnis wird also nicht nachträglich korrigiert, sondern direkt korrekt ermittelt. Wenn dagegen der Zusammenhang zwischen Messergebnis 16 und Messsignal 14 nicht unmittelbar analytisch beschreibbar ist, sondern über eine Kalibrationsfunktion, Kalibrationsmatrix oder dergleichen ermittelt werden muss, ist normalerweise die nachträgliche Korrektur des Messergebnisses möglich.

Die Pilotfrequenzen $f_{P1}$ und $f_{P2}$ sind so niedrig zu wählen, dass im Rahmen der zu erwartenden Längenänderungen $\Delta L$ für $\varphi_1$ und $\varphi_2$ Phasengänge größer n vermieden werden und somit n und m konstant bleiben. Gleiches gilt für die Differenzfrequenz bei Nutzung der Differenzphase. Andererseits können die Pilotfrequenzen $f_{P1}$ und $f_{P2}$ mittels geeigneter Werte für n bzw. m groß genug gewählt werden, um eine ausreichende Auflösung bei der Messung der Längenänderungen $\Delta L$ zu erzielen.

Es besteht auch die Möglichkeit, eine der Pilotfrequenzen, z. B. $f_{P1}$, so groß zu wählen, dass sie eine hohe Winkelauflösung gewährleistet, wobei aber die Anzahl der Vollschwingungen variieren kann. Um die Eindeutigkeit wieder herzustellen, wird die andere Pilotfrequenz $f_{P2}$ genutzt und so niedrig gewählt, dass sich die Anzahl ihrer Vollschwingungen nicht ändert und die Auflösung ausreicht, um die Anzahl der Vollschwingungen bei der höheren Pilotfrequenz $f_{P1}$ zu bestimmen. Das Prinzip ist entsprechend kaskadierbar, so dass auch kleine Längenänderungen bestimmt werden können.

In den gezeigten Ausführungsbeispielen sind im Wesentlichen nur die für die Beschreibung der Erfindung notwendigen oder hilfreichen Komponenten dargestellt. So wurde beispielsweise nicht darauf eingegangen, dass der Injektionsstrom i regelmäßig, z. B. nach jeder Abtastperiode, zusätzlich mit einem Burstsignal moduliert und das Messsignal 14 anhand der aus dem Burstsignal resultierenden Signalanteile automatisch verstärkt und normalisiert werden kann.

Das gezeigte Absorptionsspektrometer kann alternativ auf Basis der direkten Absorptionsspektroskopie arbeiten. Dabei entfallen der zweite Signalgenerator 9 und der erste Lock-in-Verstärker 17. Die Absorption wird direkt ermittelt, indem der im Wesentlichen der Form der Absorptionslinie entsprechende Verlauf des Messsignals 14 unmittelbar ausgewertet wird.

**Patentansprüche**

**1.** Absorptionsspektrometer zur Messung der Konzentration einer interessierenden Gaskomponente eines Messgases (1), mit

- einem wellenlängendurchstimmbaren Laser (4),
- einem Detektor (5) zur Erzeugung eines Messsignals (14),
- einer im Lichtweg (3) zwischen dem Laser (4) und dem Detektor (5) liegenden und das Messgas (1) enthaltenden Multireflexions-Gaszelle (2),
- Steuermitteln (6, 7, 9, 11, 13, 26) zur Variation des Injektionsstroms (i) des Lasers (4), um die Wellenlänge des Lichts auf eine spezifische Absorptionslinie der interessierenden Gaskomponente abzustimmen, und
- Auswertemitteln (15) zur Auswertung des Messsignals (14) und Ermittlung eines Messergebnisses (16) für die zu messende Konzentration,

**dadurch gekennzeichnet,**

- **dass** die Steuermittel (11) einen oder zwei Pilotsignalgeneratoren (11, 26) enthalten, um den Injektionsstrom (i) abwechselnd oder gleichzeitig mit zwei Pilotfrequenzen ($f_{P1}$, $f_{P2}$) im MHz-Bereich zu modulieren, und
- **dass** weitere Auswertemittel (21) vorhanden sind, um in dem Messsignal (14) enthaltene Signalanteile mit den Pilotfrequenzen ($f_{P1}$, $f_{P2}$) jeweils phasensensitiv auszuwerten, eine Differenz ($\Delta\varphi_{meas}$) der dabei erhaltenen Phaseninformationen ($\varphi_1$, $\varphi_2$) beider Signalanteile zu ermitteln und anhand dieser Differenz ($\Delta\varphi_{meas}$)

- die Länge ($L_{meas}$) des Lichtwegs (3) zu bestimmen, mit der aus der von dem Messsignal (14) repräsentierten Absorption das Messergebnis (16) unmittelbar ermittelt oder durch Vergleich mit einer bei Kalibration des Absorptionsspektrometers erhaltenen Länge ($L_{calib}$) oder der bekannten Länge ($L_0$) des Lichtwegs (3) in Abhängigkeit von dem Verhältnis der beiden verglichenen Längen ($L_{meas}$, $L_{calib}$ oder $L_0$) korrigiert wird, oder
- durch Vergleich mit einer bei Kalibration des Absorptionsspektrometers erhaltenen Differenz ($\Delta\varphi_{calib}$) der Phaseninformationen in Abhängigkeit von dem Verhältnis der beiden verglichenen Differenzen ($\Delta\varphi_{meas}$, $\Delta\varphi_{calib}$) das Messergebnis (16) zu korrigieren.

2. Absorptionsspektrometer nach Anspruch 1, **dadurch gekennzeichnet, dass** es auf der Basis der Wellenlängen-Modulationsspektroskopie mit einer der Pilotfrequenzen ($f_{P1}$, $f_{P2}$) als Modulationsfrequenz arbeitet, wobei die Auswertemittel (15) dazu ausgebildet sind, zur Ermittlung des Messergebnisses (16) das Messsignal (14) bei einer höheren Harmonischen ($nf_p$, $nf_{P1}$, $nf_{P2}$), insbesondere der zweiten Harmonischen ($2f_P$, $2f_{P1}$, $2f_{P2}$), der als Modulationsfrequenz verwendeten Pilotfrequenz ($f_P$, $f_{P1}$, $f_{P2}$) auszuwerten.

3. Verfahren zur Messung der Konzentration einer interessierenden Gaskomponente eines Messgases (1), wobei

- die Wellenlänge des Lichts eines Lasers (4) auf eine spezifische Absorptionslinie der interessierenden Gaskomponente abgestimmt wird,
- das modulierte Licht durch eine das Messgas (1) enthaltende Multireflexions-Gaszelle (2) auf einen Detektor (5) geführt wird und
- ein von dem Detektor (5) erzeugtes Messsignal (14) zur Ermittlung eines Messergebnisses (16) für die zu messende Konzentration ausgewertet wird,

**dadurch gekennzeichnet,**

- **dass** das Licht des Lasers (4) abwechselnd oder gleichzeitig mit zwei Pilotfrequenzen ($f_{P1}$, $f_{P2}$) im MHz-Bereich zu moduliert wird, und
- **dass** in dem Messsignal (14) enthaltene Signalanteile mit den Pilotfrequenzen ($f_{P1}$, $f_{P2}$) jeweils phasensensitiv ausgewertet werden, eine Differenz ($\Delta\varphi_{meas}$) der dabei erhaltenen Phaseninformationen ($\varphi_1$, $\varphi_2$) beider Signalanteile ermittelt wird und anhand dieser Differenz ($\Delta\varphi_{meas}$)

- die Länge ($L_{meas}$) des Lichtwegs (3) bestimmt wird, mit der aus der von dem Messsignal (14) repräsentierten Absorption das Messergebnis (16) unmittelbar ermittelt oder durch Vergleich mit einer bei Kalibration des Absorptionsspektrometers erhaltenen Länge ($L_{calib}$) oder der bekannten Länge ($L_0$) des Lichtwegs (3) in Abhängigkeit von dem Verhältnis der beiden verglichenen Längen ($L_{meas}$, $L_{calib}$ oder $L_0$) korrigiert wird, oder
- durch Vergleich mit einer bei Kalibration des Absorptionsspektrometers erhaltenen Differenz ($\Delta\varphi_{calib}$) der Phaseninformationen in Abhängigkeit von dem Verhältnis der beiden verglichenen Differenzen ($\Delta\varphi_{meas}$, $\Delta\varphi_{calib}$) das Messergebnis (16) korrigiert wird.

**4.** Verfahren nach Anspruch 3, **dadurch gekennzeichnet, dass** die eine Pilotfrequenz so hoch gewählt wird, dass sich die daraus erhaltene Phaseninformationen bei einer maximal tolerierbaren Änderung der optischen Wegstrecke zwischen dem Laser (4) und dem Detektor (5) um mehr als k·π (k ≥ 1) ändert, und die andere Pilotfrequenz so niedrig gewählt wird, dass sich die daraus erhaltene Phaseninformation bei der maximal tolerierbaren Änderung der optischen Wegstrecke um weniger als n ändert, und dass anhand der Phaseninformation aus der niedrigeren Pilotfrequenz der Wert k in der Phaseninformation aus der höheren Pilotfrequenz bestimmt wird.

**Claims**

**1.** Absorption spectrometer for measuring the concentration of a gaseous component of interest in a measurement gas (1), comprising

  - a variable-wavelength laser (4),
  - a detector (5) for generating a measurement signal (14),
  - a multi-reflection gas cell (2) situated in the light path (3) between the laser (4) and the detector (5) and containing the measurement gas (1),
  - control means (6, 7, 9, 11, 13, 26) for varying the injection current (i) of the laser (4), to tune the wavelength of the light to a specific absorption line of the gaseous component of interest, and
  - analysis (15) means for analysing the measurement signal (14) and establishing a measured result (16) for the concentration to be measured,

**characterised in that**

  - the control means (11) contain one or two pilot signal generators (11, 16) to modulate the injection current (i) alternately or simultaneously with two pilot frequencies ($f_{P1}$, $f_{P2}$) in the MHz range and
  - further analysis means (21) are present to analyse signal components contained in the measurement signal (14) with the pilot frequencies ($f_{P1}$, $f_{P2}$) in a phase-sensitive manner in each case, to establish a difference ($\Delta\varphi_{meas}$) between the items of phase information ($\varphi_1$, $\varphi_2$) of the two signal components obtained in this operation and on the basis of said difference ($\Delta\varphi_{meas}$)

  - to determine the length ($L_{meas}$) of the light path (3), with the aid of which the measured result (16) is established immediately from the absorption represented by the measurement signal (14) or is corrected by comparing with a length ($L_{calib}$) obtained during calibration of the absorption spectrometer or the known length ($L_0$) of the light path (3) as a function of the relationship between the two compared lengths ($L_{meas}$, $L_{calib}$ or $L_0$), or
  - to correct the measured result (16) by comparing with a difference ($\Delta\varphi_{calib}$) of the phase information obtained during calibration of the absorption spectrometer as a function of the relationship between the two compared differences ($\Delta\varphi_{meas}$, $\Delta\varphi_{calib}$).

**2.** Absorption spectrometer according to claim 1, **characterised in that** it functions on the basis of wavelength-modulation spectroscopy with one of the pilot frequencies ($f_{P1}$, $f_{P2}$) as the modulation frequency, wherein the analysis means (15) is realised so as to analyse the measurement signal (14) at a higher harmonic ($nf_P$, $nf_{P1}$, $nf_{P2}$), and in particular the second harmonic ($2f_P$, $2f_{P1}$, $2f_{P2}$), of the pilot frequency ($f_P$, $f_{P1}$, $f_{P2}$) used as the modulation frequency in order to establish the measured result (16).

**3.** Method for measuring the concentration of a gaseous component of interest in a measurement gas (1), wherein

  - the wavelength of the light from a laser (4) is tuned to a specific absorption line of the gaseous component of interest,
  - the modulated light is routed through a multi-reflection gas cell (2) containing the measurement gas (1) on to a detector (5), and
  - a measurement signal (14) generated by the detector (5) is analysed to establish a measured result (16) for the concentration to be measured,

**characterised in that**

  - the light from the laser (4) is modulated with two pilot frequencies ($f_{P1}$, $f_{P2}$) in the MHz range alternately or simultaneously, and

- signal components contained in the measurement signal (14) with the pilot frequencies ($f_{P1}$, $f_{P2}$) are analysed in a phase-sensitive manner in each case, a difference ($\Delta\varphi_{meas}$) between the items of phase information ($\varphi_1$, $\varphi_2$) of the two signal components obtained in this operation is established and on the basis of said difference

- the length ($L_{meas}$) of the light path (3) is determined, with the aid of which the measured result (16) is established immediately from the absorption represented by the measurement signal (14) or is corrected by comparing with a length ($L_{calib}$) obtained during calibration of the absorption spectrometer or the known length ($L_0$) of the light path (3) as a function of the relationship between the two compared lengths ($L_{meas}$, $L_{calib}$ or $L_0$), or

- the measured result (16) is corrected by comparing with a difference ($\Delta\varphi_{calib}$) of the phase information obtained during calibration of the absorption spectrometer as a function of the relationship between the two compared differences ($\Delta\varphi_{meas}$, $\Delta\varphi_{calib}$).

4. Method according to claim 3, **characterised in that** one pilot frequency is selected sufficiently high so that in the case of a maximum tolerable change in the optical path distance between the laser (4) and the detector (5), the phase information obtained in this operation changes by more than $k\cdot\pi$ ($k \geq 1$), and the other pilot frequency is selected sufficiently low so that in the case of the maximum tolerable change in the optical path distance, the phase information obtained in this operation changes by less than n, and that the value k in the phase information from the higher pilot frequency is determined on the basis of the phase information from the lower pilot frequency.

## Revendications

1. Spectromètre d'absorption pour mesurer la concentration d'un constituant gazeux auquel on s'intéresse d'un gaz (1) à mesurer, comprenant

- un laser (4) de longueur d'onde variable,
- un détecteur (5) de production d'un signal (14) de mesure,
- une cellule (2) de gaz à réflexions multiples se trouvant dans le trajet (3) de la lumière entre le laser (4) et le détecteur (5) et contenant le gaz (1) à mesurer,
- des moyens (6, 7, 9, 11, 13, 26) de commande, pour faire varier le courant (i) d'injection du laser (4), afin d'adapter la longueur d'onde de la lumière à une raie d'absorption spécifique du constituant gazeux auquel on s'intéresse et
- des moyens (15) d'exploitation, pour exploiter le signal (14) de mesure et pour déterminer un résultat (16) de mesure de la concentration à mesurer,

**caractérisé**

- **en ce que** les moyens (11) de commande comportent un ou deux générateurs (11, 26) de signal pilote, pour moduler le courant (i) d'injection, en alternance ou en même temps, par deux fréquences ($f_{p1}$, $f_{p2}$) pilotes dans le domaine MHz et
- **en ce qu'**il y a d'autres moyens (21) d'exploitation pour exploiter, d'une manière sensible en phase, par les fréquences ($f_{p1}$, $f_{p2}$) pilotes, des parties du signal contenues dans le signal (14) de mesure, pour déterminer une différence ($\Delta\varphi_{meas}$) des informations ($\varphi_1$, $\varphi_2$) de phase obtenues des deux parties du signal et, au moyen de cette différence ($\Delta\varphi_{meas}$)
- déterminer la longueur ($L_{meas}$) du trajet (3) de la lumière, par laquelle le résultat (16) de la mesure est déterminé directement à partir de l'absorption représentée par le signal (14) de mesure ou est corrigé par comparaison à une longueur ($L_{calib}$) obtenue à l'étalonnage du spectromètre d'absorption ou à la longueur ($L_0$) connue du trajet (3) de la lumière, en fonction du résultat des deux longueurs $L_{meas}$, $L_{calib}$ ou $L_0$) comparées ou
- pour corriger le résultat (16) de mesure, par comparaison à une différence ($\Delta\varphi_{calib}$), obtenue à l'étalonnage du spectromètre d'absorption, des informations de phase en fonction du rapport des deux différences ($\Delta\varphi_{meas}$, $\Delta\varphi_{calib}$) comparées.

2. Spectromètre d'absorption suivant la revendication 1, **caractérisé en ce qu'**il fonctionne sur la base de la spectroscopie par modulation de longueur d'onde, en ayant l'une des fréquences ($f_{p1}$, $f_{p2}$) pilotes comme fréquence de modulation, les moyens (15) d'exploitation étant, pour déterminer le résultat (16) de la mesure, constitués pour exploiter le signal (14) de mesure à un harmonique ($nf_p$, $nf_{p1}$, $nf_{p2}$) supérieur, notamment au deuxième harmonique

($2f_p$, $2f_{p1}$, $2f_{p2}$) de la fréquence ($f_p$, $f_{p1}$, $f_{p2}$) pilote utilisée comme fréquence de modulation.

3. Procédé de mesure de la concentration d'un constituant gazeux auquel on s'intéresse d'un gaz à mesurer, dans lequel

- on adapte la longueur d'onde de la lumière d'un laser (4) à une raie d'absorption précise du constituant gazeux auquel on s'intéresse,
- on envoie la lumière modulée sur un détecteur (5), en passant par une cellule (2) de gaz à réflexions multiples contenant le gaz (1) à mesurer et
- on exploite un signal (14) de mesure, produit par le détecteur (5), pour la détermination d'un résultat (16) de mesure de la concentration à mesurer,

**caractérisé**

- **en ce que** l'on module, dans le domaine MHz, la lumière du laser (4), en alternance ou simultanément, à deux fréquences ($f_{p1}$, $f_{p2}$) pilotes et
- **en ce que** l'on exploite, d'une manière sensible à la phase, des parties de signal contenues dans le signal (14) de mesure, par les fréquences ($f_{p1}$, $f_{p2}$) pilotes, on détermine une différence ($\Delta\varphi_{meas}$) des informations ($\varphi_1$, $\varphi_2$) de phase obtenues des deux parties du signal et, à l'aide de cette différence ($\Delta\varphi_{meas}$)
- on détermine la longueur ($L_{meas}$) du trajet (3) lumineux, par laquelle le résultat (16) de la mesure est déterminé directement à partir de l'absorption représentée par le signal (14) de mesure ou est corrigé par comparaison à une longueur ($L_{calib}$) obtenue à l'étalonnage du spectromètre d'absorption ou à la longueur ($L_0$) connue du trajet (3) de la lumière, en fonction du résultat des deux longueurs ($L_{meas}$, $L_{calib}$ ou $L_0$) comparées ou
- on corrige le résultat (16) de mesure par comparaison à une différence ($\Delta\varphi_{calib}$), obtenue à l'étalonnage du spectromètre d'absorption, des informations de phase, en fonction du rapport des deux différences ($\Delta\varphi_{meas}$, $\Delta\varphi_{calib}$) comparées.

4. Procédé suivant la revendication 3, **caractérisé en ce que** l'on choisit une fréquence pilote si haute que les informations de phase qui en sont obtenues se modifient de plus de $k\cdot\pi$ ($k \geq 1$) à une modification tolérable au maximum du trajet optique entre le laser (4) et le détecteur (5) et on choisit l'autre fréquence pilote si basse que l'information de phase qui en est obtenue se modifie de moins de n à la variation tolérable au maximum du trajet optique et **en ce que**, à l'aide de l'information de phase, on détermine la valeur k dans l'information de phase provenant de la fréquence pilote la plus haute.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5173749 A